# EUROPEAN PATENT APPLICATION

(11) **EP 4 295 773 A1**
(43) Date of publication of application: **27.12.2023**
(21) Application number: 22180753.0
(22) Date of filing: 23.06.2022
(51) Int. Cl.: A61B 6/02, A61B 6/03, A61B 6/00

(54) **CT IMAGING METHOD**

(71) Applicant: Koninklijke Philips N.V., 5656 AG Eindhoven (NL)
(72) Inventor: GRASS, Michael, Eindhoven (NL); KOEHLER, Thomas, Eindhoven (NL); PROKSA, Roland, 5656AG Eindhoven (NL)
(74) Representative: Philips Intellectual Property & Standards

(57) **Abstract**

A method for cone beam Computed Tomography (CT) imaging in which an axial scan is performed, and wherein a helical scan is performed immediately afterwards, starting from the same plane of rotation.

## Description

### FIELD OF THE INVENTION

The present invention relates to the field of Computed Tomography (CT) imaging, and in particular to cone beam CT imaging.

### BACKGROUND OF THE INVENTION

Axial cone beam Computed Tomography (CT) imaging can be applied clinically to achieve full organ coverage at high temporal resolution for organs e.g. the heart or the brain. Axial imaging means performing a sequence of axial scans at consecutive axial positions along the body. An axial scan means a scan formed from a partial or complete rotation of the CT generator and detector at a fixed position of the body along the (longitudinal) axial direction. Axial cone beam imaging offers several advantages such as high temporal resolution scanning, spatial and temporal coherence of the data set and efficient multi-phase imaging.

However, axial cone beam imaging also has some disadvantages. One important disadvantage is the presence of cone beam artefacts in reconstructed image data. These appear with increasing distance from the plane of source rotation.

Other cone beam CT methods also exist. One is cone beam helical imaging. This comprises a helical motion of the detector and radiation source along a cranial or caudal direction relative to the object(s) being scanned. This method reduces cone beam artefacts because the helical motion smoothly acquires data from all possible planes. However, in particular the temporal coherence is necessarily reduced as a consequence.

Developments in the field of cone beam CT imaging are generally sought.

### SUMMARY OF THE INVENTION

The invention is defined by the claims.

According to examples in accordance with an aspect of the invention, there is provided a method for cone beam Computed Tomography (CT) imaging, the method comprising controlling execution of a scan protocol for a CT scanner comprising an x-ray source and detector mounted at diametrically opposite locations on a rotatable gantry, the gantry rotatable about a support platform which is axially displaceable through a central examination region, the scan protocol comprising performing an axial scan of a first scan volume, the axial scan comprising rotation of the gantry at a fixed axial position with a fixed plane of rotation; performing a helical scan of a further scan volume starting from the said plane of rotation of the axial scan, the helical scan comprising rotation of the gantry about a changing axial position; wherein, the axial scan and the helical scan are performed temporally contiguously, without any time interval between the two; and wherein, the first scan volume and further scan volume partially overlap.

Embodiments of the invention thus propose a dual-mode scanning protocol comprising an axial/circular scan followed by a helical scan, wherein imaged volumes of the two overlap, and wherein the helical scan starts from the axial scan plane of the axial scan. This has the benefit that the acquired projection data for the two scans can be combined during reconstruction to reduce the cone beam artefacts in the axial scan reconstruction, and/or to increase the spatial resolution of the helical scan reconstruction in the region where the helical and axial scan volumes overlap.

The concept of performing an axial and helical scan as a part of a single, continuous, uninterrupted scanning procedure/protocol has not previously been suggested. Typically, a CT scanning apparatus will have a set of scan protocols which can be selected between, where these may include axial and helical scans. However, these are fully independent scan operations, and involve a data acquisition and image reconstruction phase. Moving from one scan protocol to the next requires significant resetting time of the scanner, and cannot be done instantly. It has never before been considered to construct a dedicated scanning protocol or control operation which involves temporally contiguous and overlapping axial and helical scans. In preferred embodiments, the method comprises no pause for a reconstruction step between the axial and helical scans.

The helical scan may comprise a variable-pitch helical scan phase, and a fixed-pitch helical scan phase; wherein the variable-pitch helical scan phase takes place between the axial scan and the fixed-pitch helical scan phase; and in the variable-pitch scan phase, the pitch of the helical scan increases towards the fixed-pitch of the fixed-pitch helical scan phase.

The pitch of the helical scan is defined as the table distance travelled in one 360 degree gantry rotation, optionally further divided by beam collimation (which is usually called "relative pitch").

The variable-pitch helical scan phase represents a transition from the axial scan to the fixed-pitch helical scan phase. Thus, the variable-pitch scan phase may be understood as a transition phase, during which the patient support platform is being accelerated from stationary to the necessary speed to realize the fixed-pitch of the fixed-pitch scan phase. During the transition phase, the support platform speed is increasing.

Higher pitch reduces the time taken to perform the helical part of the scan, which is advantageous for reducing the likelihood of any motion of a scanned object, e.g. a patient, during the procedure. Thus, this allows the temporal resolution of the scan to remain high.

The fixed-pitch of the fixed-pitch helical scan phase may correspond to an axial displacement speed of the support platform relative to the gantry of at least half the detector collimation per rotation. Preferably a larger pitch is used, e.g. 1.4 or even 1.6 times the detector collimation. Additionally or alternatively, the axial displacement speed of the support platform may be at least 400 mm/s or more preferably 600 mm/s.

The variable-pitch of the variable-pitch helical scan phase may correspond to an axial acceleration of the support platform relative to the gantry of at least 600 mm/s² or more preferably at least 800 mm/s².

The fixed-pitch of the fixed-pitch helical scan phase may correspond to a rotation rate of the gantry of at least 150 rpm, more preferably at least 200 rpm, more preferably at least 240 rpm.

The speed and acceleration of the support platform relative to the gantry in part facilitates the combination of the data of the two scans. This is because there will be less chance of motion of a scanned object, e.g. a patient, during the transition between the two, and this also allows the temporal resolution to remain sufficiently high to be of clinical interest.

In some embodiments, the axial scan may comprise a rotation of the detector and x-ray source through at least 180 degrees. This allows an axial scan to be performed in less time than if it were to rotate a full 360 degrees which may not be necessary depending on the aim of the scan.

The axial scan may comprise rotation of the detector and x-ray source through at least 360 degrees. This allows a more comprehensive axial scan, but also requires more time to execute.

The method may further comprise forming a composite projection dataset comprising the whole of the projections dataset for one of the axial and helical scan, and a portion of the projection data of the other of the axial and helical scan corresponding to an overlapping portion of the first and second scan volumes. This allows data from the helical scan acquisition to be used to reduce cone beam artefacts in the axial scan, or vice versa.

The composite projection dataset may comprise the whole of the projection dataset of both the axial and helical scan. This can improve noise reduction in the combined images and may remove other types of artefacts, e.g. metal artefacts.

In some embodiments, the method may further comprise reconstructing the composite projection dataset to form reconstructed image data.

The reconstruction of the composite projection dataset may comprise use of a direct inversion reconstruction method. The reconstruction of the composite projection dataset may comprise use of a second pass reconstruction method. The reconstruction of the composite projection dataset may comprise use of an iterative reconstruction method. The reconstruction of the composite projection dataset may comprise use of an AI-based reconstruction method.

The method in alternative embodiments may comprise first reconstructing the projection dataset for the axial scan and reconstructing the projection dataset for the helical scan, and subsequently applying a neural network trained to generate a single final image based on the reconstructed datasets for the axial scan and the helical scan.

A further aspect of the invention also provides a processing arrangement for use in Computed Tomography (CT) imaging. The processing arrangement comprises an input/output for operably coupling to a CT scanning apparatus, the CT scanning apparatus comprising a x-ray source and a detector mounted at diametrically opposite locations on a rotatable gantry. The processing arrangement further comprises one or more processors adapted to communicate control commands to the CT scanner via the input/output to control execution of a scan protocol comprising performing an axial scan of a first scan volume, the axial scan comprising rotation of the gantry at a fixed axial position and with a fixed plane of rotation; performing a helical scan of a further scan volume starting from the said plane of rotation of the axial scan, the helical scan comprising rotation of the gantry about a changing axial position; wherein, the axial scan and the helical scan are performed temporally contiguously, without any time interval between the two; and wherein, the first scan volume and further scan volume partially overlap.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a schematic illustration of an imaging system such as a CT scanner;
Fig. 2 shows a schematic illustration of exemplary detector and/or radiation source trajectories for an axial scan, a variable-pitch helical scan, and a fixed-pitch helical scan;
Fig. 3 shows a schematic view of an acquisition strategy for a cardiac imaging case with the axial scan region, the helical scan region, and the joint volume region;
Fig. 4 shows a flow diagram of one exemplary method of combining the datasets of both the axial and helical scans to produce one artefact free image; and
Fig. 5 shows a schematic diagram of a processing arrangement for use in Computed Tomography imaging.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for cone beam Computed Tomography (CT) imaging in which an axial scan, otherwise known as a circular scan, is performed, and then a helical scan is performed immediately afterwards without any time interval between them. The circular scan comprises rotating the gantry at a fixed axial position with a fixed plane of rotation. The helical scan comprises starting from the same plane of rotation as the circular scan and then rotating the gantry about an increasing axial position relative to a scanned object(s). The data from these scans can then be combined in a plurality of ways to reduce cone beam artefacts in either or both of them. For example, during reconstruction of the circular scan, data from the overlapping portion of the helical scan may be combined with the circular scan dataset to reduce cone beam artefacts that would have arisen otherwise. Similarly, during reconstruction of the helical scan, data from the overlapping portion of the circular scan may be combined with the helical scan dataset to reduce cone beam artefacts. In another embodiment, the entirety of the datasets of the circular scan and the helical scan may be combined to produce one reconstructed image with improved noise reduction and reduction of cone beam artefacts in the overlapping portion, as well as the reduction of other types of artefacts, e.g. metal artefacts.

Data from both helical and axial scans starting from the same plane of rotation could be combined in order to reduce cone beam artefacts among other types of artefact.

In other words, the method consists of the acquiring of projection data of a circular partial scan or full scan of a target organ of interest, and then acquiring projection data of a helix scan starting from the circular plane of source rotation and then moving in a cranial or caudal direction.

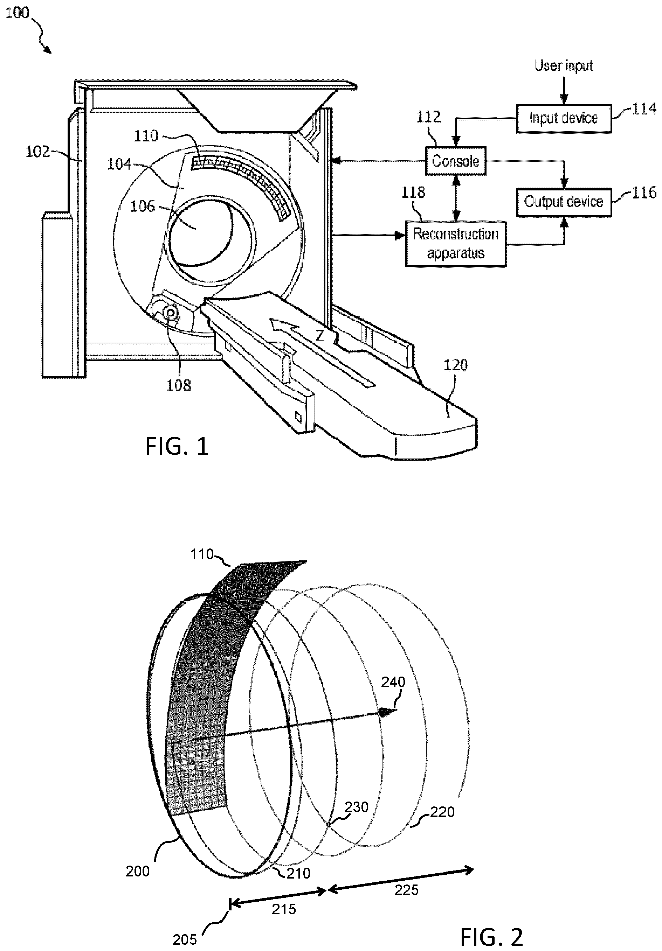

Fig. 1 schematically illustrates an imaging system 100 such as a CT scanner. The imaging system 100 includes a generally stationary gantry 102 and a rotatable gantry 104, which is rotatably supported by the stationary gantry and rotates around an examination region 106 about a z-axis. A subject support 120, such as a couch, supports an object or subject in the examination region.

A radiation source 108, such as an x-ray tube, is rotatably supported by the rotatable gantry 104, rotates with the rotatable gantry, and emits radiation that traverses the examination region 106. In one instance, the radiation source includes a single broad spectrum x-ray tube. In another instance, the radiation source includes a single x-ray tube configured to switch between at least two different emission voltages (e.g., 80 kVp and 140 kVp) during scanning. In yet another instance, the radiation source includes two or more x-ray tubes configured to emit radiation having different mean spectra. In still another instance, the radiation source includes a combination thereof.

A radiation sensitive detector array 110 subtends an angular arc opposite the radiation source 108 across the examination region 106. The radiation sensitive detector array detects radiation traversing the examination region and generates an electrical signal(s) (projection data) indicative thereof. The radiation sensitive detector array may include an energy-resolving detector (e.g., direct conversion photon counting detectors, at least two sets of scintillators with different spectral sensitivities (multi-layer), etc.). The detector array can include a single layer detector, a direct conversion photon counting detector, and/or a multi-layer detector. The direct conversion photon counting detectors may include a conversion material such as CdTe, CdZnTe, Si, Ge, GaAs, or other direct conversion material. An example of multi-layer detector includes a double decker detector such as the double decker detector described in US 7,968,853 B2.

A reconstruction apparatus 118 receives non-spectral or spectral projection data from the detector array 110 and reconstructs volumetric image data such as sCCTA image data, a high-energy image, a low energy image, a photoelectric image, a Compton scatter image, an iodine image, a calcium image, a virtual non-contrast image, a bone image, a soft tissue image, and/or other basis material image. The reconstruction apparatus can also reconstruct non-spectral volumetric image data, e.g., by combining spectral projection data and/or spectral volumetric image data. In case of spectral projection data and/or spectral volumetric image data, this will include data for at least two different energies and/or energy ranges.

A console 112 serves as an operator console. The console is operably connected to an output device 116 such as a monitor and an input device 114 such as a keyboard, mouse, etc. Software resident on the console 112 allows the operator to interact with and/or operate the imaging system 100 via a graphical user interface (GUI) or otherwise.

The present invention provides a method for cone beam CT imaging, the method comprising controlling execution of a scan protocol for a CT scanner, such as the one shown in Fig. 1, comprising an x-ray source 108 and detector 110 mounted at diametrically opposite locations on a rotatable gantry 104, the gantry rotatable about a support platform 120 which is axially displaceable through a central examination region 106.

The scan protocol executed by the CT scanner comprises performing an axial scan 200 of a first scan volume, the axial scan comprising rotation of the gantry 104 at a fixed axial position with a fixed plane of rotation; and performing a helical scan of a further scan volume starting from the said plane of rotation of the axial scan, the helical scan comprising rotation of the gantry around a changing axial position. The axial scan and the helical scan are preferably performed temporally contiguously, without any time interval between the two. The first scan volume and further scan volume partially overlap. Having both scans start from the same said plane of rotation and be performed temporally contiguously facilitates efficient combination of data from either scan with the other in the overlapping scan volume.

In some embodiments, the axial scan may comprise rotation of the detector 110 and x-ray source 108 through at least 180 degrees. This allows a quicker axial scan when compared to a full 360 degree axial scan, the latter of which may not be necessary depending on the aim of the scan. In some embodiments, the axial scan comprises rotation of the detector and x-ray source through at least 360 degrees. This allows a more comprehensive axial scan, but also requires more time to execute.

Fig. 2 shows a schematic illustration of a rotation path of the detector and radiation source about the examination region 106 according to an example scan protocol executed in accordance with one or more embodiments of the invention.

Loop 200 illustrates a circular rotation path of the detector and x-ray source for the axial scan. The combination of helixes 210 and 220 illustrate the helical rotation path of the x-ray source 108 and detector 110 for the helical scan. As will be apparent, the helical scan path follows continuously from the end of the axial scan path.

As illustrated in Fig. 2, in some embodiments, the helical scan comprises a first 210 and second 220 helical scan phase. The first phase is a variable-pitch helical scan phase and the second phase is a fixed-pitch helical scan phase. Point 230 shows the point at which the variable-pitch helical phase ends and the fixed-pitch helical phase begins. The pitch of a helical scan may be defined as the patient support platform 120 distance travelled in one 360 degree gantry rotation. Alternatively, the pitch of a helical scan may be defined as the patient support platform distance travelled in one 360 degree gantry rotation, divided by beam collimation.

The variable-pitch helical scan phase 210 takes place temporally and spatially between the axial scan 200 and the fixed-pitch helical scan phase 220. In the variable-pitch scan phase, the pitch of the helical scan increases towards the fixed-pitch of the fixed-pitch helical scan phase.

The variable-pitch helical scan phase 210 is a transition phase from the axial scan 200 to the fixed-pitch helical scan phase 220, in which the patient support 120 must accelerate from a speed of zero to a speed needed for the fixed-pitch helical scan phase.

In some embodiments, the fixed-pitch of the fixed-pitch helical scan phase 220 may correspond to an axial displacement speed of the support platform 120 relative to the gantry 104 of at least 400 mm/s or more preferably at least 600 mm/s. Additionally or alternatively, the axial displacement speed of the support platform may be at least half the detector collimation per rotation. More preferably a larger pitch is used, e.g. 1.4 or even 1.6 times the detector collimation.

The patient support 120 acceleration during the variable-pitch phase 210 is preferably as high as possible, as this reduces the time needed to perform the overall scan protocol, and so reduces the likelihood of any motion of a scanned object, e.g. a patient, during the procedure and keeps the temporal resolution high.

By way of example, in some embodiments, the variable-pitch of the variable-pitch helical scan phase corresponds to an axial acceleration of the support platform relative to the gantry 104 of at least 600 mm/s² or at least 700 mm/s², or at least 800 mm/s².

The speed and acceleration of the support platform 120 relative to the gantry 104 allows efficient combination of the data of the two scans as there will be less chance of motion of a scanned object, e.g. a patient, during the procedure, and the temporal resolution can remain sufficiently high to be of clinical interest.

Since it is important for the acquisitions to achieve good temporal resolution, it is important to note that a support platform 120 that offers up to 800 mm/s² acceleration speed allows a combined axial and helical scan to yield approx. 440 msec additional data acquisition time (e.g. 1.7 full gantry rotations at 240 RPM rotation speed) compared to only an axial scan. This is the time it takes for the source to leave the axial scan volume towards the cranial or caudal direction.

Specifically, additional data is acquired during table acceleration for the overlapping scan volume, allowing enhanced reduction of cone beam artefacts in the overlapping volume.

The whole scan protocol comprises an axial displacement of the gantry 104 relative to the patient support 120 along the axial path 240. The total axial displacement for the variable-pitch helical phase is indicated at 215. The total axial displacement for the fixed-pitch helical phase is shown at 225. For the axial scan, there is no axial displacement of the gantry relative to the patient support, and so the rotation path 200 of the axial scan defines a single plane of rotation for the axial scan, whose position is schematically indicated at 205. As will be apparent, the helical scan 210, 220 begins at this plane of rotation of the axial scan.

Although the axial scan comprises only a single plane of rotation, the scan volume for which projection data is acquired in the axial scan has an axial width due to the width of the cone beam. Thus the axial scan acquires scan data for a short cylindrical volumetric scan region. This scan volume for the axial scan partially overlaps with the beginning portion (the trailing end) of the scan volume which is covered by the helical scan. In particular it partially overlaps with the scan volume for the variable-pitch helical scan.

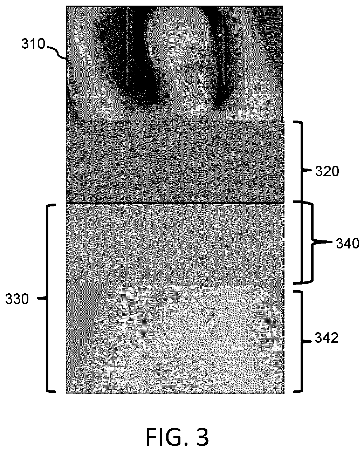

The overlap of the scan volumes for the different scan phases is better illustrated in Fig. 3.

Fig. 3 shows a schematic view of the scan volumes acquired by the scan protocol in accordance with an example cardiac imaging scan of a patient 310. The scan begins at the chest region with the axial scan, the axial scan effectively corresponding to a scan pitch of zero. The axial scan acquires CT projection data for a scan volume 320 of a width determined by the width of the cone beam. The helical scan follows immediately after the axial scan, beginning at the rotation plane of the axial scan. The helical scan acquired CT projection data for a scan volume. The helical scan volume includes volume portions 340, 342 corresponding respectively to the variable-pitch helical scan phase 210, and the fixed-pitch helical scan phase 220. A region of overlap between the scan volumes and 330 of the axial scan and the helical scan respectively may be referred to as a joint volume region. In this example, the joint volume region corresponds to the whole of the scan volume of the variable-pitch helical scan, and one portion of the scan volume of the axial scan. This can be understood from the fact that the variable-pitch helical scan phase begins at the same rotation plane as the axial scan, but immediately begins to move axially away from this scan plane. Thus the overlap between these scan volumes can be expected to be only a portion of the total volume covered by the axial scan. It is noted that this is just one example, and in other cases the overlapping volume region may correspond to just a portion of the variable-pitch scan volume.

The overlapping scan volume is the region for which data from the helical scan and the axial scan may be used in combination in reconstruction, to thereby reduce cone beam artefacts and/or image noise. In other words, the portion of the axial scan projection data and helical scan projection data which corresponds to the overlapping scan volume region can be used in combination to form a final image reconstruction. This will now be described in greater detail.

There are at least two main ways to combine the scan data for the axial 200 and helical scans 210,220 to form the final reconstruction. One approach is to perform a data combination or fusing operation in the projection domain, wherein at least the portion of the projection data from the axial scan and helical scan corresponds to the overlapping scan volume region is combined to form a composite projection dataset, and then this composite projection dataset is reconstructed with a reconstruction algorithm. A second approach is to perform the combining in the image domain by first separately reconstructing an image dataset for the axial and helical scans, and then feeding these as respective inputs to a trained neural network which is trained to generate a final output image volume which has reduced artefacts.

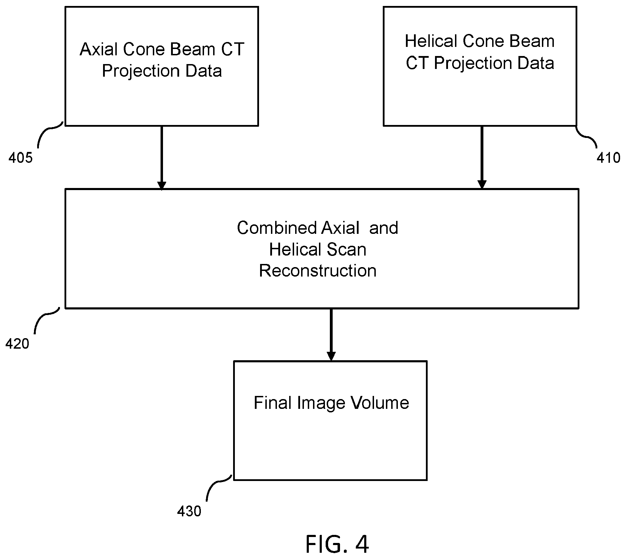

Taking the first approach, in some embodiments, the method may further comprise forming a composite projection dataset comprising the whole of the dataset for one of the axial 200 and helical scan 210,220 and a portion of the projection data of the other of the axial and helical scan corresponding to an overlapping portion 340 of the first and second scan volumes. With reference to Fig. 4, the method comprises receiving 405 the projection data from the axial cone beam CT scan and receiving 410 the projection data from the variable pitch helical cone beam CT scan from the CT scanner. The projection data from both scans is then reconstructed 420, for example, using an aperture weighted wedge method (as described in detail in US6775346B2). This results in a respective volumetric image 430.

This allows data acquired from the helical scan 210,220 to be used to improve the reconstruction of the overlapping portion 340 of the axial scan 200 and to reduce cone beam artefacts that appear with increasing distance from the plane of source rotation. Alternatively, the data from the axial scan can be used to reduce cone beam artefacts in the overlapping region of the helical scan.

The reconstruction algorithm applied to the helical scan data may be an approximate reconstruction in some examples (at least in the most widespread used filtered back projection formulation) and thus the additional projections available from the axial scan 200 may also help to reduce the cone beam artefacts in the reconstructed helical scan images.

In some embodiments, the composite projection dataset may comprise the whole of the projection dataset of both the axial 200 and helical scan 210,220. This allows the improvement of noise reduction in the combined image and may remove other types of artefacts, e.g. metal artefacts.

In the case of joint reconstruction of the whole of both data sets, an AI denoising neural network may be used which includes spatial input patches from both volumes and uses them in joint denoising. In some embodiments, there may further be a step of compensating for the motion of the scanned object or patient during the scan protocol. Methods for this are known in the art. One example may be the use of an AI neural network which has been trained to perform image registration prior to image denoising.

In some embodiments, the reconstruction of the composite projection dataset may comprise use of a direct inversion reconstruction method. In some embodiments, the reconstruction of the composite projection dataset may comprise use of a second pass reconstruction method. In some embodiments, the reconstruction of the composite projection dataset may comprise use of an AI-based reconstruction method. In some embodiments, the reconstruction of the composite projection dataset may comprise use of an iterative reconstruction method.

In some embodiments, the method may further comprise reconstructing the projection dataset for the axial scan and reconstructing the projection dataset for the helical scan, and subsequently applying a neural network trained to generate a single final image based on the reconstructed datasets for the axial scan and the helical scan.

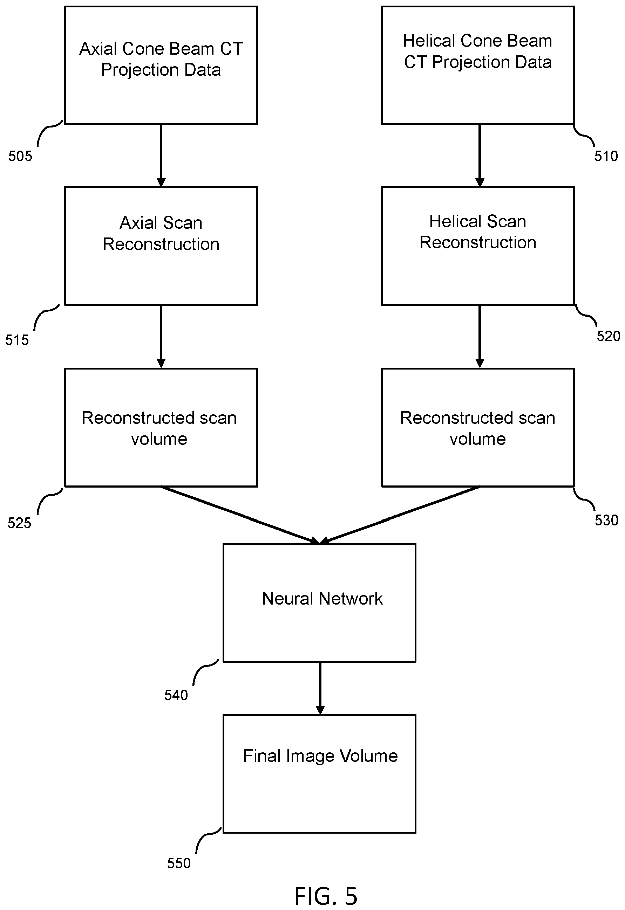

Fig. 5 shows a flow diagram of one exemplary method of combining the datasets of both the axial and helical scans to produce one artefact free image. With reference to Fig. 5, the method comprises receiving 505 the projection data from the axial cone beam CT scan and receiving 510 the projection data from the variable pitch helical cone beam CT scan from the CT scanner. The projection data from the axial cone beam CT scan is then reconstructed 515, for example, using an aperture weighted wedge method, and the projection data from the variable pitch helical cone beam CT scan is reconstructed 520, for example using an aperture weighted wedge method (as described in detail in US6775346B2). This results in a respective volumetric image 525 for the axial scan, and a respective volumetric image 530 for the helical scan. A trained neural network is then applied 540 to the two volume image data sets to produce 550 a final image volume, with reduced cone beam artefacts compared to the input scan data.

Optionally, denoising may be applied to each reconstructed image, e.g. using the AI denoising neural network mentioned above.

Above have been mentioned use of neural networks. The training of the neural network may comprise using training data generated from simulations based on mathematical phantoms and/or physical phantoms or patient images. These will then be forward projected in the desired system geometry using the desired trajectory.

Reference has been made to a neural network, but any machine learning algorithm may be suitable. A machine-learning algorithm is any self-training algorithm that processes input data in order to produce or predict output data.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

Methods for the reconstruction of each of the projection datasets are well known in the art.

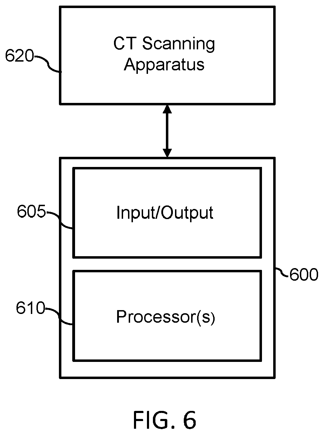

Fig. 6 shows a schematic diagram of a processing arrangement 600 for use in Computed tomography (CT) imaging, the processing arrangement comprising:
an input/output 605 for operably coupling to a CT scanning apparatus 620, the CT scanning apparatus comprising a x-ray source and a detector mounted at diametrically opposite locations on a rotatable gantry, and one or more processors 610 adapted to communicate control commands to the CT scanner via the input/output to control execution of a scan protocol comprising performing an axial scan of a first scan volume, the axial scan comprising rotation of the gantry at a fixed axial position and with a fixed plane of rotation; and performing a helical scan of a further scan volume starting from the said plane of rotation of the axial scan, the helical scan comprising rotation of the gantry about a changing axial position. The axial scan and the helical scan are performed temporally contiguously, without any time interval between the two, and the first scan volume and further scan volume partially overlap.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A method for cone beam Computed Tomography (CT) imaging, the method comprising:
controlling execution of a scan protocol for a CT scanner comprising an x-ray source (108) and detector (110) mounted at diametrically opposite locations on a rotatable gantry (104), the gantry rotatable about a support platform (120) which is axially displaceable through a central examination region (106), the scan protocol comprising:
performing an axial scan (200) of a first scan volume, the axial scan comprising rotation of the gantry (104) at a fixed axial position with a fixed plane of rotation;
performing a helical scan (210,220) of a further scan volume starting from the said plane of rotation of the axial scan, the helical scan comprising rotation of the gantry about a changing axial position (240);
wherein, the axial scan and the helical scan are performed temporally contiguously, without any time interval between the two; and
wherein, the first scan volume and further scan volume partially overlap.

2. The method of claim 1, wherein
the helical scan comprises: a variable-pitch helical scan phase (210), and a fixed-pitch helical scan phase (220);
the variable-pitch helical scan phase (210) takes place between the axial scan (200) and the fixed-pitch helical scan phase (220); and
in the variable-pitch scan phase (210), the pitch of the helical scan increases towards the fixed-pitch of the fixed-pitch helical scan phase (220).

3. The method of claim 2, wherein the fixed-pitch of the fixed-pitch helical scan phase corresponds to an axial displacement speed of the support platform relative to the gantry of at least 400-600 mm/s, preferably at least 500 mm/s, or even more preferably at least 600 mm/s.

4. The method of claim 2 or 3, wherein the variable-pitch of the variable-pitch helical scan phase corresponds to an axial acceleration of the support platform relative to the gantry of at least 600-800 mm/s², preferably at least 700 mm/s², or even more preferably at least 800 mm/s².

5. The method of any of claims 1-4, wherein the axial scan comprises rotation of the detector and x-ray source through at least 180 degrees.

6. The method of any of claims 1-5, wherein the axial scan comprises rotation of the detector and x-ray source through at least 360 degrees.

7. The method of any of claims 1-6, wherein the method further comprises forming a composite projection dataset comprising the whole of the projection dataset of one of the axial and helical scan, and a portion of the projection dataset of the other of the axial and helical scan corresponding to an overlapping portion of the first and second scan volumes.

8. The method of any of claim 7, wherein the composite projection dataset comprises the whole of the projection dataset of both the axial and helical scan.

9. The method of claim 7 or 8, wherein the method further comprises reconstructing the composite projection dataset to form reconstructed image data.

10. The method of any of claims 9, wherein the reconstruction of the composite projection dataset comprises use of a direct inversion reconstruction method.

11. The method of claim 9, wherein the reconstruction of the composite projection dataset comprises use of a second pass reconstruction method.

12. The method of claim 9, wherein the reconstruction of the composite projection dataset comprises use of an iterative reconstruction method.

13. The method of claim 9, wherein the reconstruction of the composite projection dataset comprises use of a trained neural network.

14. The method of any of claims 1-6, wherein the method further comprises reconstructing the projection dataset for the axial scan and reconstructing the projection dataset for the helical scan, and subsequently applying a neural network trained to generate a single final image based on the reconstructed datasets for the axial scan and the helical scan.

15. A processing arrangement (600) for use in Computed Tomography (CT) imaging, the processing arrangement comprising:
an input/output (605) for operably coupling to a CT scanning apparatus (620), the CT scanning apparatus comprising a x-ray source and a detector mounted at diametrically opposite locations on a rotatable gantry; and
one or more processors (610) adapted to communicate control commands to the CT scanner via the input/output to control execution of a scan protocol comprising:
performing an axial scan of a first scan volume, the axial scan comprising rotation of the gantry at a fixed axial position and with a fixed plane of rotation;
performing a helical scan of a further scan volume starting from the said plane of rotation of the axial scan, the helical scan comprising rotation of the gantry about a changing axial position;
wherein, the axial scan and the helical scan are performed temporally contiguously, without any time interval between the two; and
wherein the first scan volume and further scan volume partially overlap.
